# EUROPEAN PATENT SPECIFICATION

(11) **EP 2 208 724 B1**
(45) Date of publication and mention of the grant of the patent: **07.12.2011**
(21) Application number: 09180333.8
(22) Date of filing: 22.12.2009
(51) Int. Cl.: C07C 405/00

(54) **Process for purification of latanoprost, synthetic analogue of prostaglandin PGF 2alfa**
Verfahren zur Reinigung von Latanoprost
Procédé pour la purification du latanoprost

(30) Priority: 24.12.2008 IT MI20082330
(43) Date of publication of application: 21.07.2010
(73) Proprietor: INDUSTRIALE CHIMICA S.r.l., 20145 Milano (IT)
(72) Inventor: Costantino, Francesca, 20147, Milano (IT); Di Brisco, Roberto, 28069, TRECATE (IT)
(74) Representative: Palladino, Saverio Massimo

(56) References cited:
- WO-A-02/096898
- WO-A-2006/112742

## Description

The present invention refers to a process for the purification of Latanoprost, a synthetic analogue of prostaglandin PGF₂ₐ, by removal in particular of the isomers of the compound produced during synthesis of the same. Latanoprost, together with newer active principles such as Travoprost and Bimatoprost, plays a key role on the market of products used to control the progression of glaucoma.

Latanoprost, with the systematic IUPAC name isopropyl-(Z)-7-[(1*R*,2*R*,3*R*,5*S*)-3,5-dihydroxy-2-[(3*R*)3-hydroxy-5-phenylpentyl]-cyclopentyl]hept-5-enoate (also referred to in literature as 13,14-dihydro-17-phenyl-18,19,20-trinor-PGF_{2α}-isopropyl ester) has the following structural formula (1):

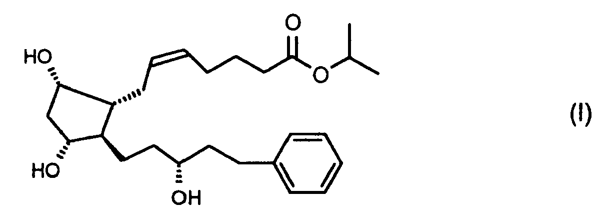

This molecule has configuration R in position 15 (which carries the hydroxyl) and cis to the double bond between positions 5 and 6.

A synthesis strategy of easy industrial applicability for this group of prostaglandins consists of variations of Corey's synthesis (Corey et al., J. Am. Chem. Soc, (1969), 5675; Corey et al., J. Am. Chem. Soc. 92, (1970), 2586; Corey et al., Tetrahedron Letters 307, 1970); the synthesis is also described in patents EP 0364417 B9 and US 6,689,901 B2 and in patent application WO 2002/096898 A2.

This synthesis approach, variously modified, leads however to the formation of impurities among which three isomers of Latanoprost, the 15-(S)-5,6-cis isomer, the 15-(R)-5,6-trans isomer and the 15-(S)-5,6-trans isomer, having the following formulas (II), (III) and (IV) respectively:

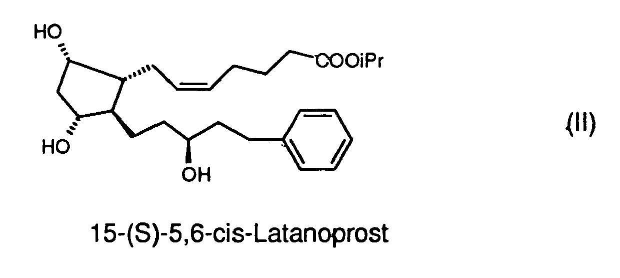

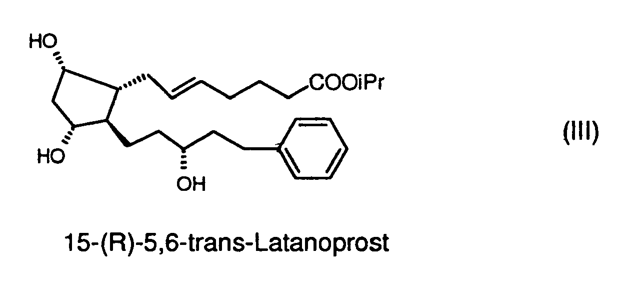

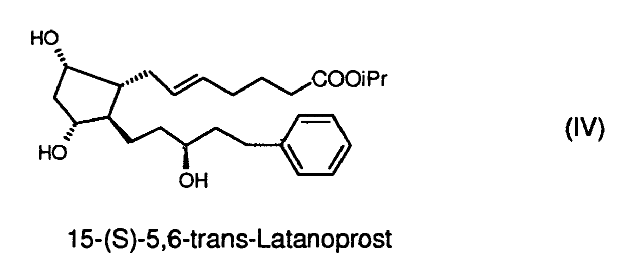

The formation of these three diastereoisomeric impurities during the synthesis of the product of interest cannot be eliminated, and the same are always present in significant quantities in the Latanoprost produced, although the quantity varies according to the reagents and the protective groups used during the synthesis.

For therapeutic use, contamination by impurities with unknown pharmacological activity, including the diastereoisomers of the active ingredient, must be minimised. The end product purification process is therefore of decisive importance in the synthesis process.

Example 5 of the patent EP 0364417 89 describes the purification of the reaction product, consisting of a mixture of alcohols epimeric at carbon-15 represented by the following formula: 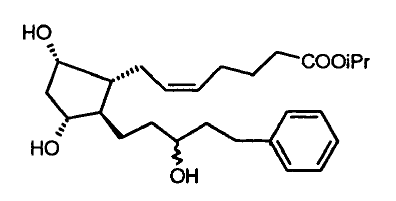 by means of preparative liquid chromatography using as eluant 40% v/v of acetonitrile in water. This reverse phase preparative HPLC method is difficult to apply on an industrial scale as it involves considerable amounts of aqueous eluant which then have to be extracted with large quantities of organic solvents to recover the product.

More recent documents describe various direct phase chromatographic methods for the purification of Latanoprost.

Patent EP 0544899 B1 describes the use of two successive columns in silica gel with two different eluants (diisopropylether/acetone; methylene chloride/isopropanol) with overall yield of 55.5%.

Patent US 6,927,300 B2 describes purification by chromatography on silica gel with hexane/ethyl acetate to obtain Latanoprost having ¹H NMR compatible with the data in literature.

Patent US 6,689,901 B2 describes purification by means of gravimetric chromatography in methyltertbutyl ether (ratio raw product/silica gel 230-400 mesh: 1/150).

Patent application EP 1721894 A1 describes the use of a gravimetric column on silica gel in methyltertbutyl ether to isolate the finished product for the purposes of spectroscopic characterisation.

Patent application US 2005/0261374 A1 describes and claims as purification process, in particular from the the three isomers of Latanoprost, a method of liquid chromatography in HPLC using as eluant a mixture consisting of one or more hydrocarbons, one or more alcohols and, optionally, acetonitrile. In the best separation conditions identified in this application (example 13c) the following relative retention times were recorded:

| **Product** | **Relative retention time** |
|---|---|
| Latanoprost | 1.00 |
| 15-(S)-5,6-cis isomer | 0.95 |
| 15-(S)-5,6-trans isomer | 1.13 |
| 15-(R)-5,6-trans isomer | 1.23 |

The eluant mixture used is heptane:isopropanol:acetonitrile in volume percentage ratios 93%:4%:3% on WaterS^{RTM} Spherisorb Silica column, but according to the text of the application, analogous results can be obtained with Phenomenex^{RTM} Luna Cyano and Phenomenex^{RTM} Luna Silica columns. The purification with Phenomenex^{RTM} Luna Silica stationary phase was verified by the Applicant, on both an analytical and preparatory scale, observing high retention times.

The purification process of US 2005/0261374 A1 requires large amounts of elution solvent as the product begins to elute at 50 minutes and elutes for 50 minutes; 10 litres of solvent per gram of pure product recovered are therefore required. A further difficulty in the purification method of this document lies in the elution order. The isomer 15(S)-cis (relative retention time 0.95) is eluted before the Latanoprost, while the other two isomers, 15(S)-trans and 15(R)-trans (with relative retention times of 1.13 and 1.23) are eluted after the Latanoprost. A separation of this type requires a high fractionation, i.e. the collection of a large number of fractions, and control analysis of each fraction is necessary before re-combining it with the others. Furthermore, the purification method of US 2005/0261374 A1 requires the use of a ternary mixture of solvents; this makes difficult the recovery of the mixture via distillation, a necessary condition for reducing the costs of the purification process via chromatograpy on preparative HPLC column. The recovery of a mixture, in fact, also presupposes an analytical support for control of the percentage content of the distillation fractions.

For these reasons the need is still felt for a Latanoprost purification process, purifying it in particular of the three isomers 15-(S)-5,6-cis, 15-(R)-5,6-trans and 15-(S)-5,6-trans, which is inexpensive and easy to apply on an industrial scale.

The subject of the present invention is therefore a process for purifying Latanoprost of its diastereoisomers 15-(S)-5,6-cis, 15(R)-5,6-trans and 15-(S)-5,6-trans, comprising the following steps:
a) obtaining Latanoprost according to a known synthesis method;
b) gravimetric chromatography on the product of step a);
c) purification of the product of step b) by means of high pressure liquid chromatography in a single eluant;
d) filtering on silica of the product of step c) with a mixture of an alcohol and a hydrocarbon;
e) filtering on membrane of the product of step d), first as a solution in an organic solvent and then as pure oil.

Characteristics and advantages of the invention will be illustrated in detail in the following description, with reference to the figures, in which:
- figure 1 shows the chromatographic profile of the Latanoprost obtained with the process of the invention;
- figure 2 shows the chromatographic profile of a sample without Latanoprost.

The inventors have found that purification of the Latanoprost by preparative HPLC can be performed, with commercially available stationary phase, using a single solvent as eluant of the chromatography, permitting the implementation of a high pressure liquid chromatography process which is economically competitive and actually industrially applicable compared to analogous known processes.

The first step of the process of the invention, a), consists in the production of Latanoprost with processes known in literature, preferably according to the synthesis method illustrated in example 3 of patent EP 0364417 B9, which should be referred to for details of the synthesis.

The second step of the process, b), consists in performing a gravimetric chromatography on silica gel of the product of step a); the eluant used in this step can be chosen among methyl acetate, ethyl acetate or, preferably, isopropyl acetate. The product of step b) will be indicated below also as "raw product".

Step c) consists in a purification by preparative high pressure liquid chromatography (HPLC) performed using as eluant a pure solvent chosen among ethyl ether, isopropyl ether, tetrahydrofuran or, preferably, methyltertbutyl ether. The stationary phase used consists of silica gel having the following characteristics: particle dimension between 10 and 15 µm, diameter of pores between 80 and 100 Å and surface area between 300 and 500 m²/g; preferred values of these characteristics are particle dimension between 11 and 13 µm, pore diameter between 90 and 96 Å and surface area between 380 and 420 m²/g.

The flow of solvent in elution is between 200 ml/min and 1000 ml/min, and preferably of about 400 ml/min; the elution temperature is between 15 °C and 30 °C, preferably between 20 °C and 25 °C.

The weight ratio between raw product and silica gel of the HPLC column is between 1/150 and 1 /100 and preferably 1/115. Operating in these conditions, the minimum number of chromatographies that can be performed before having to regenerate the silica is at least 30.

The product of step c) is eluted at a concentration of between 0.68 g/l and 0.76 g/I; operating according to the above preferred conditions, said concentration is between 0.70 g/I and 0.74 g/I.

In step c) the components are eluted in the following order: Latanoprost, isomer 15(R)-trans, isomer 15(S)-cis and isomer 15(S)-trans. The fact that Latanoprost is the first component eluted constitutes an undoubted advantage as it eliminates the double fractionation both at the head and tail of the product.

The quantity of pure Latanoprost recovered in step c) is between 50% and 70% of the theoretical amount; operating in the preferred conditions described above, said quantity is between 55% and 65% of the theoretical amount.

The fraction eluted containing the Latanoprost (i.e. the first fraction collected coming out of the column) then undergoes a filtering step d) on silica with a mixture of an alcohol and a hydrocarbon; examples of mixtures that can be used for the purpose are mixtures of an alcohol chosen among methanol, ethanol, 2-propanol and tert-butanol and a hydrocarbon chosen among n-hexane, n-heptane, cyclohexane, toluene and mixtures of isomers of hexane and heptane; the preferred mixture is 2-propanol/n-heptane. In particular the ratio in volume between the alcohol and the hydrocarbon is between 50:50 and 1:99 and preferably between 30:70 and 3:97.

Lastly, the product of step d) undergoes step e), which consists in double filtering on a membrane, the first as a solution in an organic solvent chosen among ethanol, methanol, ethyl ether, isopropyl ether, methyltertbutyl ether and, preferably, acetone; the second membrane filtering is performed on the Latanoprost as pure oil. In particular the filtering degree of the membranes used is between 0.65 pm and 0.1 µm and preferably between 0.45 pm and 0.22 µm.

In a preferred embodiment of the process of the invention, step c) also comprises two further steps, c') and c"), which are performed after step c) and before step d). These two steps consist in:
c') rechromatography of the unsuitable fractions from point c), still containing product but enriched with the three diastereoisomeric impurities, by means of the same high pressure liquid chromatography as point c);
c") recovery of the solvent by distillation.

In step c') the fractions recovered after the first one, enriched in Latanoprost, still containing non-negligible percentages of the compound of interest, are recombined and undergo a further purification cycle according to the procedure of step c). Via this operation it is possible to recover a further 20% of Latanoprost with respect to the theoretical content of compound contained in the raw product. In principle, it would be possible to further recycle in preparative HPLC the "waste" fractions of step c'), but after the first recycle the advantages in terms of quantity of product recoverable do not justify the costs of the operation.

In step c") the solvent recovered from step c) (or c')) is recycled by means of simple distillation. Distillation rectification columns or analytical control on the content of the distillate are not necessary. It is therefore possible to perform a total recycle of the eluant used for the purification, thus obtaining a reduction in process costs.

The invention will be further illustrated by means of the following example.

### EXAMPLE

This example refers to purification of raw Latanoprost by means of preparative HPLC. All the percentages given in the example are measured as areas of the bands of the UV spectrum relative to the various isomers; measurement wavelength: 210 nm.

A sample of Latanoprost obtained according to the process described in EP 0364417 B9 and containing 4.6% of the isomer 15-(S)-5,6-cis, 5.9% of the isomer 15(R)-5,6-trans and 0.42% of the isomer 15-(S)-5,6-trans, is filtered on packed silica and eluted with isopropyl acetate.

9.4 g of raw Latanoprost recovered as described above (HPLC titre: 68%) are dissolved in 100 ml of methyltertbutyl ether pre-distilled at atmospheric pressure and are chromatographed in the following conditions:
- preparative HPLC: LaPrep System VWR
- eluant: distilled methyltertbutyl ether (purity GC ≥ 99%; water content ≤0.2%)
- 100 mm column packed with 1.150 kg of PHENOMENEX LUNA 15µ SILICA (2) 100 Å
- flow of 400 ml/min
- total elution time 65 minutes
- process control by UV detector at 215 nm.

The solvent (3.6 l) is eliminated by distillation until a constant weight of the oil is reached, resulting in 5 g of Latanoprost (clear oil).

The remaining fraction of solvent eluted and containing Latanoprost and impurities is concentrated by distillation until a constant weight is obtained (4.4 g, yellow oil). This residue is rechromatographed according to the procedures described above. 1.8 g of Latanoprost (clear oil) are obtained.

The two Latanoprost samples are filtered on 270 g of silica gel using a mixture formed of 95 parts of n-heptane and 5 parts of 2-propanol (parts in volume).

The residue obtained, after distillation of the solvent, is dissolved in pure acetone, filtered on membrane (0.22 µ; GV Millipore) and concentrated in a vacuum at 40 ± 5 °C.

Lastly, the oil obtained is filtered on a membrane (0.45 p; HVHP Millipore).

6 grams of pure Latanoprost (in the form of a clear oil) are obtained with a content of each of the above three isomers below 0.1 %.

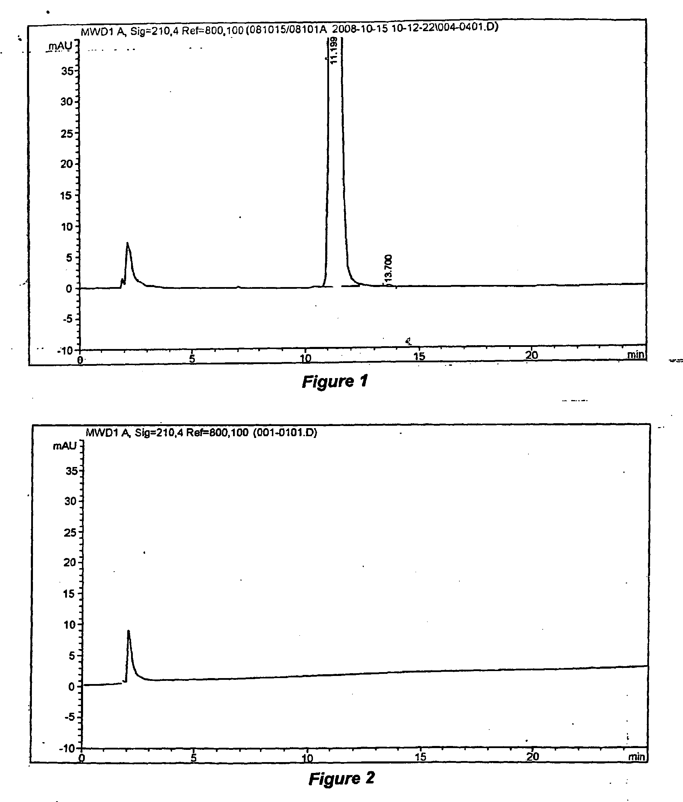

The chromatographic profile of the Latanoprost obtained is shown in figure 1. Attribution to Latanoprost of the intense peak at a retention time between approximately 11 and 12 minutes is done by performing a comparative test with the solvent only, without Latanoprost (the result is shown in figure 2); the solvent peak is determined at a retention time of approximately 2 minutes.

### Comments

Compared to the known purification methods, the process of the present invention offers several advantages.

Firstly, in the preparative HPLC step c), one single fraction of eluate is obtained containing the three diastereoisomers of the Latanoprost, as "tails" of the compound of interest, which can be recycled according to the same chromatographic method as used in step c). In the methods of the known art, on the other hand, fractions are obtained enriched in a different way in the three different impurities, which cannot be re-chromatographed with the same method as the one used for the first chromatographic purification, entailing considerable difficulties in recovery of the parts of Latanoprost contained in these further fractions.

Furthermore, the process of the invention requires the use of a single solvent instead of a mixture of solvents, thus avoiding various problems that occur with the use of binary or ternary mixtures in the known art. These problems are in particular the unbalance of the ratio of solvents at different boiling point during their recovery via distillation, and the need to use distillation rectification columns or analytical control on the content of the distillate before it can be re-used as an eluant for the preparative HPLC. Vice versa, in step c") of the process of the invention, the eluant is totally recovered by means of simple distillation and it is therefore possible to recycle it in the purification, obtaining a reduction in process costs.

## Claims

1. A process for the purification of isopropyl-(Z)-7-[(1*R*,2*R*,3*R*,5*S*)-3,5-dihydroxy-2-[(3*R*)3-hydroxy-5-phenylpentyl]-cyclopentyl]hept-5-enoate, having the following structural formula: 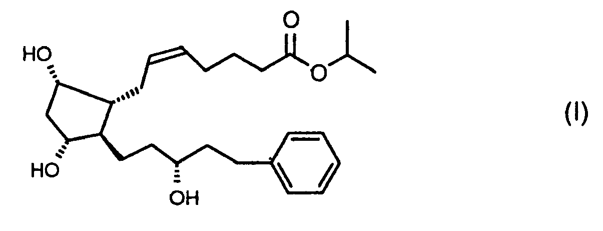 purifying it of its diastereoisomers having the following structural formulas: 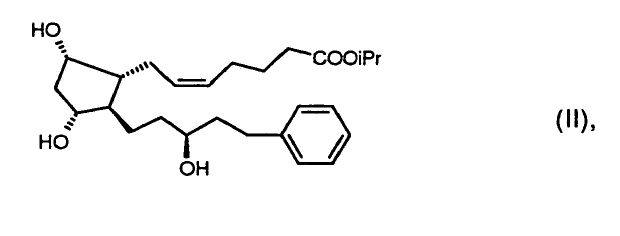 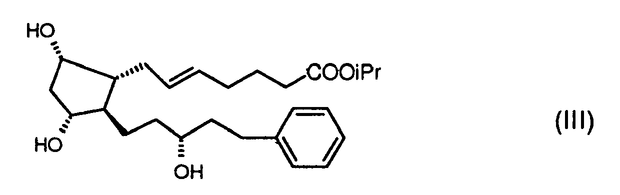 and 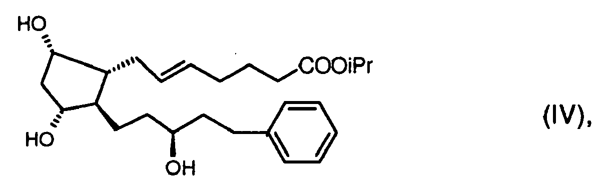 said process comprising the following steps:
a) obtaining Latanoprost according to a known synthesis method;
b) gravimetric chromatography on the product of step a);
c) purification of the product of step b) by means of high pressure liquid chromatography in a single eluant;
d) filtering on silica of the product of step c) with a mixture of an alcohol and a hydrocarbon;
e) filtering on membrane of the product of step d), first as a solution in an organic solvent and then as pure oil.

2. Process according to claim 1 further comprising the two additional following steps c') and c"), performed after step c) and before step d):
c') rechromatography of the unsuitable fractions from point c), still containing product but enriched with the three diastereoisomeric impurities, by means of the same high pressure liquid chromatography as point c);
c") recovery of the solvent by means of distillation and re-use of the same.

3. Process according to one of claims 1 or 2 wherein the content of said diastereoisomers in the end product is reduced to a value below 0.1 %.

4. Process according to claim 1 wherein step b) is performed by using as eluant an organic ester chosen among methyl acetate, ethyl acetate and isopropyl acetate.

5. Process according to claim 1 wherein the solvent used as eluant in step c) is chosen among ethyl ether, isopropyl ether, tetrahydrofuran and methyltertbutyl ether.

6. Process according to claim 1 wherein the mixture between an alcohol and a hydrocarbon of step d) is formed of an alcohol chosen among methanol, ethanol, 2-propanol and tert-butanol and a hydrocarbon chosen among n-hexane, n-heptane, cyclohexane, toluene and mixtures of isomers of hexane and heptane.

7. Process according to claim 6 wherein said mixture is between 2-propanol and n-heptane.

8. Process according to claim 1 wherein the organic solvent used in step e) is chosen among ethanol, methanol, ethyl ether, isopropyl ether, methyltertbutyl ether and acetone.

9. Process according to claim 8 wherein said solvent is acetone.

10. Process according to claim 8 wherein the Latanoprost is filtered on a membrane as a solvent-free product.

## Patentansprüche

1. Verfahren zur Reinigung von Isopropyl-(Z)-7-[(1R, 2R, 3R, 5S)-3,5-dihydroxy-2-[(3R)3-hydroxy-5-phenylpentyl]-cyclopentyl]hept-5-enoat mit der nachfolgenden Strukturformel: 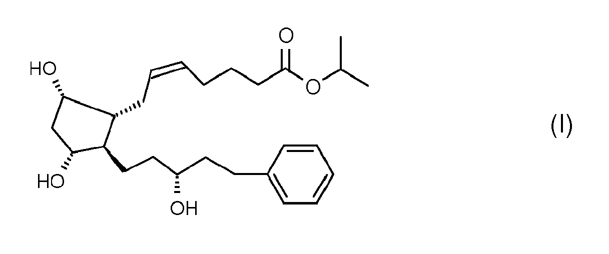 durch Reinigen desselben von den Diastereoisomeren mit den nachfolgenden Strukturformeln: 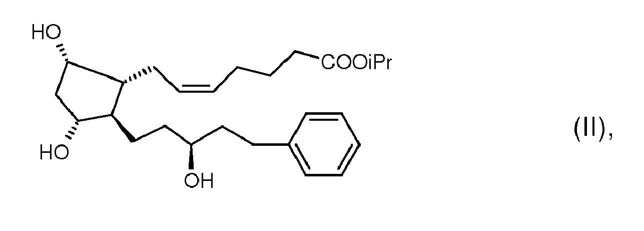 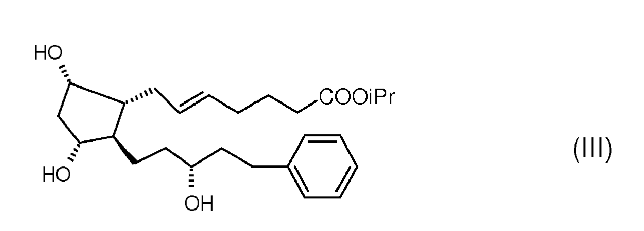 und 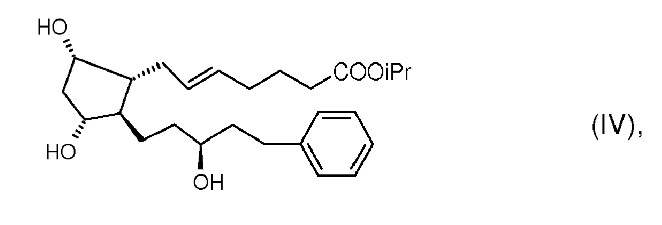 wobei das Verfahren die nachfolgenden Schritte umfasst:
a) Bereitstellen von Latanoprost nach einem bekannten Syntheseverfahren,
b) gravimetrische Chromatographie des Produkts aus dem Schritt a),
c) Reinigen des Produktes aus dem Schritt b) mittels Hochleistungsflüssigkeitschromatographie in einem einzigen Eluent,
d) Filtrieren des Produkts aus dem Schritt c) auf Kieselsäuregel mit einer Mischung aus einem Alkohol und einem Kohlenwasserstoff,
e) Filtrieren des Produkts aus dem Schritt d) auf einer Membran, und zwar zunächst als eine Lösung in einem organischen Lösungsmittel und dann als reines Öl.

2. Verfahren nach Anspruch 1, welches des weiteren die beiden nachfolgenden Schritte c') und c") umfasst, welche nach dem Schritt c) und vor dem Schritt d) durchgeführt werden:
c') Rechromatographie der ungeeigneten Fraktionen von dem Schritt c), welche immer noch Produkt enthalten, das aber mit den drei Diastereoisomeren angereicht ist, durch dieselbe Hochleistungsflüssigkeitschromatographie wie in dem Schritt c),
c") Wiedergewinnen des Lösungsmittels durch Destillation und Wiederverwendung desselben.

3. Verfahren nach Anspruch 1 oder 2, wobei die Menge der Diastereoisomere in dem Endprodukt auf einen Wert von weniger als 0,1 % verringert wird.

4. Verfahren nach Anspruch 1, wobei der Schritt b) durch Verwenden eines organischen Esters ausgewählt von Methylacetat, Ethylacetat und Isopropylacetat als Eluent durchgeführt wird.

5. Verfahren nach Anspruch 1, wobei das in dem Schritt c) als Eluent verwendete Lösungsmittel aus Ethylether, Isopropylether, Tetrahydrofuran und Methyltertbutylether ausgewählt wird.

6. Verfahren nach Anspruch 1, wobei die Mischung aus einem Alkohol und einem Kohlenwasserstoff in dem Schritt d) aus einem Alkohol, welcher aus Methanol, Ethanol, 2-Propanol und tert-Butanol ausgewählt wird, sowie einem Kohlenwasserstoff, der aus n-Hexan, n-Heptan, Cyclohexan, Toluol und Mischungen von Isomeren aus Hexan und Heptan ausgewählt wird, gebildet wird.

7. Verfahren nach Anspruch 6, wobei die Mischung aus 2-Propanol und n-Heptan besteht.

8. Verfahren nach Anspruch 1, wobei das in dem Schritt e) verwendete organische Lösungsmittel aus Ethanol, Methanol, Ethylether, Isopropylether, Methyltertbutylether und Aceton ausgewählt wird.

9. Verfahren nach Anspruch 8, wobei das Lösungsmittel Aceton ist.

10. Verfahren nach Anspruch 8, wobei das Latanoprost auf einer Membran als ein lösungsmittelfreies Produkt filtriert wird.

## Revendications

1. Procédé de purification du (Z)-7-[(1*R*,2*R*,3*R*,5*S*)-3,5-dihydroxy-2-[(3-*R*)3-hydroxy-5-phénylpentyl]-cyclopentyl]hept-5-énoate d'isopropyle, ayant la formule structurelle suivants : 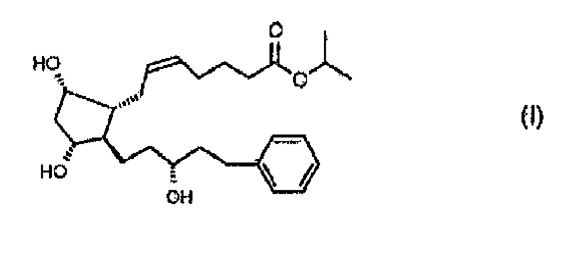 en le purifiant de ses diastéréoisomères ayant les formules structurelles suivantes : 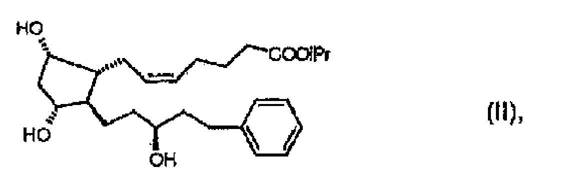 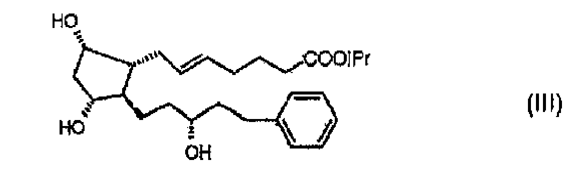 et 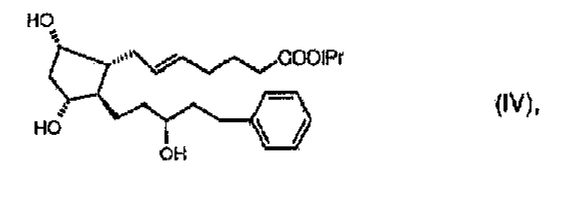 ledit procédé comprenant les étapes suivantes:
a) obtention de Latanoprost selon un procédé de synthèse connu ;
b) chromatographie gravimétrique sur le produit de l'étape a) ;
c) purification du produit de l'étape b) au moyen d'une chromatographie liquide haute pression dans un éluant unique ;
d) filtration sur silice du produit de l'étape c) avec un mélange d'un alcool et d'un hydrocarbure ;
e) filtration sur membrane du produit de l'étape d), tout d'abord en tant que solution dans un solvant organique, puis en tant qu'huile pure.

2. Procédé selon la revendication 1, comprenant en outre les deux étapes additionnelles c') et c") effectuées après l'étape c) et avant l'étape d) :
c') rechromatographie des fractions inappropriées issues de l'étape c), contenant encore du produit, mais enrichies avec les trois impuretés diastéréoisomères, au moyen de la même chromatographie liquide haute pression utilisée à l'étape c) ;
c") récupération du solvant par distillation et ré-utilisation de ce solvant.

3. Procédé selon l'une quelconque des revendications 1 ou 2, dans lequel la teneur desdits diastéréoisomères dans le produit final est réduite à une valeur en-dessous de 0,1 %.

4. Procédé selon la revendication 1, dans lequel l'étape b) est effectuée à l'aide, en tant qu'éluant, d'un ester organique choisi parmi l'acétate de méthyle, l'acétate d'éthyle et l'acétate d'isopropyle.

5. Procédé selon la revendication 1, dans lequel le solvant utilisé comme éluant à l'étape c) est choisi parmi l'éther éthylique, l'éther isopropylique, le tétrahydrofurane et l'éther tert-butyl méthylique.

6. Procédé selon la revendication 1, dans lequel le mélange d'un alcool et d'un hydrocarbure à l'étape d) est formé d'un alcool choisi parmi le méthanol, l'éthanol, le 2-propanol et le tert-butanol et d'un hydrocarbure choisi par le n-hexane, le n-heptane, le cyclohexane, le toluène et des mélanges d'isomères d'hexane et d'heptane.

7. Procédé selon la revendication 6, dans lequel ledit mélange est un mélange de 2-propanol et de n-heptane.

8. Procédé selon la revendication 1, dans lequel ledit solvant organique utilisé à l'étape e) est choisi parmi l'éthanol, le méthanol, l'éther éthylique, l'éther isopropylique, l'éther tert-butyl méthylique et l'acétone.

9. Procédé selon la revendication 8, dans lequel ledit solvant est l'acétone.

10. Procédé selon la revendication 8, dans lequel le Latanoprost est filtré sur une membrane en tant que produit exempt de solvant.
